# EUROPÄISCHE PATENTSCHRIFT

(11) **EP 2 430 971 B1**
(45) Veröffentlichungstag und Bekanntmachung des Hinweises auf die Patenterteilung: **31.08.2016**
(21) Anmeldenummer: 11175055.0
(22) Anmeldetag: 22.07.2011
(51) Int. Cl.: A61B 1/00, G02B 26/08, G02B 23/24

(54) **Endoskop mit variabler Blickrichtung**
Endoscope with variable view angle
Endoscope à direction d'observation variable

(30) Priorität: 17.09.2010 DE 102010040990
(43) Veröffentlichungstag der Anmeldung: 21.03.2012
(73) Patentinhaber: Henke-Sass, Wolf GmbH, 78532 Tuttlingen (DE)
(72) Erfinder: Pauli, Sabine, 78532 Tuttlingen (DE); Rehe, Oliver, 78532 Tuttlingen (DE)
(74) Vertreter: Patentanwälte Geyer, Fehners & Partner mbB

(56) Entgegenhaltungen:
- DE-A1- 19 839 188
- DE-B3-102009 049 143
- US-A- 3 856 000
- US-A1- 2010 030 031

## Beschreibung

Die vorliegende Erfindung betrifft ein Endoskop mit variabler Blickrichtung. Solche Endoskope weisen am distalen Ende des Endoskopschafts häufig ein schwenkbar gelagertes Umlenkelement als Teil der Abbildungsoptik auf, um mittels der Schwenkstellung des Umlenkelementes die gewünschte Blickrichtung einzustellen.

Da bei solchen Endoskopen aufgrund des Verschwenkens des Umlenkelementes der Bereich zwischen dem Umlenkelement und einem dem Umlenkelement nachfolgenden Optikelement der Abbildungsoptik vergrößert oder verkleinert wird, tritt bei einer feststehenden Blende bei verschiedenen Schwenkstellungen unerwünschtes Streulicht auf, das die Abbildungsqualität der Abbildungsoptik verschlechtert.

Es ist bekannt, eine separat betätigbare Blende vorzusehen, deren Position verändert werden kann, um in Abhängigkeit der eingestellten Schwenkstellung dann die gewünschte Blendenposition einzustellen. Dies führt jedoch zu zeitlichen Verzögerungen, da nach Einstellung der gewünschten Blickrichtung stets noch die Blendenposition nachgeführt werden muß.

Aus der US 3,856,000 ist ein Endoskop mit dem Merkmalen des Oberbegriffs des Anspruches 1 bekannt.

Ausgehend hiervon ist es Aufgabe der Erfindung, ein Endoskop mit variabler Blickrichtung bereitzustellen, bei dem bei Änderung der Blickrichtung eine effektive Streulichtunterdrückung vorliegt.

Die Aufgabe wird durch ein Endoskop mit den Merkmalen des Anspruches 1 gelöst.

Da das distale Ende des Schiebeelements den Abschirmungsabschnitt aufweist, wird zeitgleich mit Einstellung der Blickrichtung auch der Abschirmungsabschnitt und somit die Blende zur Unterdrückung von unerwünschtem Streulicht positioniert, so daß ohne Zeitverzögerung das Endoskop bei Änderung der Blickrichtung benutzt werden kann. Insbesondere ist kein manuelles Nachführen der Blendenposition nach Einstellung einer Blickrichtung notwendig.

Da das Schiebeelement als Rohr ausgebildet ist (es kann dann z. B. auch als Zugrohr bezeichnet werden), ist die Herstellung des Endoskops erleichtert.

Bevorzugt greift das distale Ende des Schiebeelementes zum Verschwenken des Umlenkelementes in einem Bereich an, der nicht auf der Schwenkachse des Umlenkelementes liegt. Ferner kann zur Kopplung des distalen Endes des Schiebeelementes mit dem Umlenkelement ein Betätigungszapfen als erstes Verbindungselement und eine den Betätigungszapfen führende Aufnahme als zweites Verbindungselement vorgesehen sein, wobei eines der beiden Verbindungselemente am Umlenkelement und das andere der beiden Verbindungselemente am Schiebeelement vorgesehen ist. Bevorzugt ist der Betätigungszapfen am Umlenkelement bzw. am Halter, in dem das Umlenkelement positioniert sein kann, vorgesehen.

Insbesondere kann das Schiebeelement das Umlenkelement antreiben, so daß eine einfach zu realisierende mechanische Lösung vorliegt, bei der synchron mit Einstellung der Blickrichtung der Abschirmungsabschnitt zur Unterdrückung von Streulicht positioniert wird.

Das Umlenkelement kann in einem drehbar gelagerten Halter befestigt sein, wobei die Drehachse des Halters bevorzugt senkrecht zur Längsrichtung des Endoskopschaftes liegt. Der Halter kann mittels des Schiebeelementes um seine Drehachse gedreht werden. Dazu kann das proximale Ende des Schiebeelementes mittels des Betätigungselementes axial bewegt werden. Insbesondere kann das Betätigungselement über ein erstes Getriebe mit dem Schiebeelement mechanisch verbunden sein. Das erste Getriebe kann bevorzugt so ausgebildet sein, daß es eine Drehbewegung des Betätigungselementes in eine axiale Bewegung des Schiebeelementes umsetzt.

Das distale Ende des Schiebeelementes weist den Abschirmungsabschnitt auf, der eine Streulichtblende bildet, die unabhängig von der eingestellten Schwenkstellung des Halters des Umlenkelementes sicherstellt, daß kein unerwünschtes Streulicht zwischen dem Umlenkelement und dem nachfolgenden Optikelement in der Abbildungsoptik gelangt.

Dazu ist das distale Ende des Schiebeelementes bevorzugt direkt mit dem Halter des Umlenkelementes mechanisch gekoppelt.

Die erste Markierungsblende kann mechanisch mit dem Betätigungselement so gekoppelt sein, daß sie synchron mit einer Änderung der Schwenkstellung des Umlenkelementes bewegt wird und mittels ihrer sichtbaren Lage bei Betrachtung des Bildes die eingestellte Blickrichtung anzeigt. Somit kann auf einfache Art und Weise im Bild immer die eingestellte Blickrichtung dargestellt werden. Einem Benutzer kann daher bei Betrachtung des Bildes stets auch visuell die Information bereitgestellt werden, welche Blickrichtung gerade eingestellt ist.

Das Betätigungselement ist bevorzugt drehbar am Handgriff gelagert. Insbesondere ist es drehbar um die Längsachse des Endoskopschaftes gelagert. Dies erleichtert die Bedienung des Endoskopes.

Ferner kann bei dem erfindungsgemäßen Endoskop der Endoskopschaft drehbar am Handgriff gelagert sein und das Endoskop eine zweite Markierungsblende aufweisen, die drehfest mit dem Endoskopschaft verbunden ist und die mittels ihrer sichtbaren Lage bei Betrachtung des Bildes die Drehstellung des Endoskopschaftes anzeigt. Somit werden in vorteilhafter Weise dem Betrachter visuell sowohl die Blickrichtung des Endoskopes als auch die Drehstellung des Endoskopschaftes dargeboten, wenn er das Bild der Abbildungsoptik betrachtet.

Insbesondere kann über die Lage der ersten Markierungsblende relativ zur Lage der zweiten Markierungsblende bei Betrachtung des Bildes die eingestellte Blickrichtung angezeigt werden. So kann z.B. der Winkelabstand der beiden Markierungsblenden variiert werden, um die Blickrichtung anzuzeigen. Insbesondere kann der Winkelabstand der beiden Markierungsblenden der eingestellten Blickrichtung (beispielsweise bezogen auf die Längsrichtung des Endoskopschaftes) entsprechen. Somit ist für den Betrachter in einfachster Art und Weise zu erfassen, welche Blickrichtung gerade eingestellt ist.

Bei dem erfindungsgemäßen Endoskop kann das Betätigungselement mit der ersten Markierungsblende über ein Untersetzungsgetriebe verbunden sein. Damit wird in vorteilhafter Weise erreicht, daß die Bewegung (beispielsweise Drehung) der ersten Markierungsblende geringer ist als die entsprechende Bewegung des Betätigungselementes.

Das Untersetzungsgetriebe kann eine Drehung des Betätigungselementes in eine axiale Bewegung und die axiale Bewegung in eine Drehbewegung der ersten Markierungsblende umsetzen. Dies kann mechanisch mit der gewünschten Genauigkeit leicht hergestellt werden.

Bevorzugt ist die erste Markierungsblende im Bereich der Feldblende der Abbildungsoptik positioniert. Auch die zweite Markierungsblende kann bevorzugt im Bereich der Feldblende der Abbildungsblende positioniert sein.

Die erste Markierungsblende ist bevorzugt relativ zur Abbildungsoptik drehbar gelagert.

Insbesondere kann die erste Markierungsblende an einer drehbar gelagerten Hülse, die z.B. koaxial zur Längsrichtung bzw. -achse des Endoskopschaftes angeordnet ist, ausgebildet sein.

Das erfindungsgemäße Endoskop kann ein Endoskop mit einem starren Endoskopschaft sein. Es ist jedoch auch möglich, daß der Endoskopschaft zumindest abschnittsweise abwinkelbar ausgebildet ist.

Das distale Ende des Endoskopschaftes kann mit einem Deckglas verschlossen sein. Insbesondere kann das distale Ende hermetisch dicht verschlossen sein, so daß der Endoskopschaft autoklavierbar ist.

Bei dem schwenkbaren Umlenkelement handelt es sich bevorzugt um einen Umlenkspiegel oder ein Umlenkprisma.

In dem Endoskopschaft ist bevorzugt noch ein Beleuchtungskanal vorgesehen, über den das abzubildende Objekt beleuchtet werden kann. Die Beleuchtung kann beispielsweise mittels Lichtleitfasern erfolgen, die am Handgriff mit Licht beaufschlagt werden können. Natürlich sind auch andere Arten der Beleuchtung möglich. Insbesondere kann eine Lichtquelle (z.B. eine oder mehrere LED-Dioden) am distalen Ende des Endoskopschaftes zur Beleuchtung vorgesehen sein.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Perspektivdarstellung einer Ausführungsform des erfindungsgemäßen Endoskopes;
- Fig. 2: eine vergrößerte Schnittdarstellung des distalen Endes des Endoskopschaftes von Fig. 1;
- Fig. 3: eine vergrößerte Seitenansicht des distalen Endes des Endoskopschaftes mit einer ersten Schwenkstellung des Umlenkprismas;
- Fig. 4: eine Draufsicht des distalen Endes des Endoskopschaftes gemäß Fig. 3;
- Fig. 5: eine vergrößerte Seitenansicht des distalen Endes des Endoskopschaftes mit einer zweiten Schwenkstellung des Umlenkprismas;
- Fig. 6: eine Draufsicht des distalen Endes des Endoskopschaftes gemäß Fig. 5;
- Fig. 7: eine vergrößerte Schnittansicht des proximalen Endes des Handgriffes des Endoskops gemäß Fig. 1;
- Fig. 8: und 9 Darstellungen zur Erläuterung der festen Markierungsblende;
- Fig. 10: und 11 Darstellungen zur Erläuterung der festen und drehbaren Markierungsblende;
- Fig. 12: eine vergrößerte Schnittdarstellung des rechten Abschnitts von Fig. 7 ohne Betätigungselement;
- Fig. 13: eine Darstellung zur Erläuterung der festen und drehbaren Markierungsblende;
- Fig. 14: eine perspektivische Explosionsdarstellung der beiden Markierungshülsen;
- Fig. 15: eine schematische Ansicht einer Variante der festen Markierungshülse;
- Fig. 16: eine schematische Darstellung einer Variante der drehbaren Markierungshülse, und
- Fig. 17: eine schematische Ansicht zur Erläuterung der Art der Darstellung der beiden Markierungsblenden bei Betrachtung des Bildes der Abbildungsoptik.

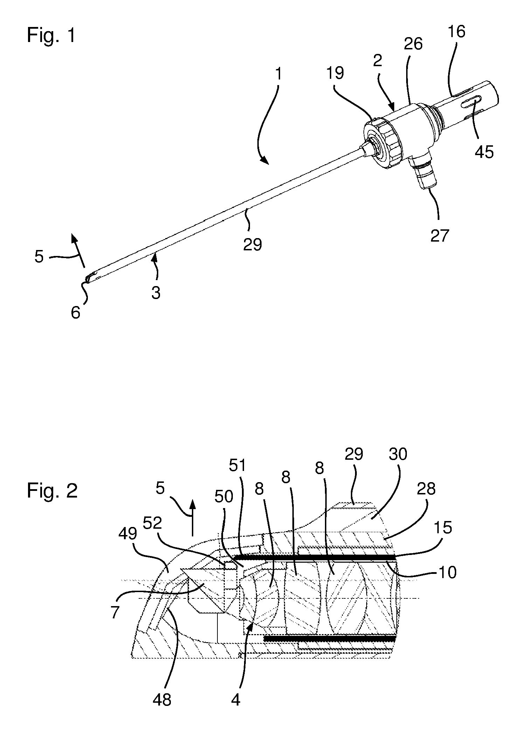

Bei der in Fig. 1 gezeigten Ausführungsform umfaßt das erfindungsgemäße Endoskop 1 mit variabler Blickrichtung einen Handgriff 2 sowie einen mit dem Handgriff 2 verbundenen Endoskopschaft 3, dessen Mantelrohr 29 in Fig. 1 sichtbar ist.

Wie insbesondere der vergrößerten Schnittdarstellung des distalen Endes 6 des Endoskopschaftes 3 in Fig. 2 zu entnehmen ist, ist im Endoskopschaft 3 ist eine Abbildungsoptik 4 angeordnet, mit der ein in Blickrichtung 5 der Abbildungsoptik 4 vor dem Endoskopschaft 3 befindliches Objekt als Bild abgebildet werden kann.

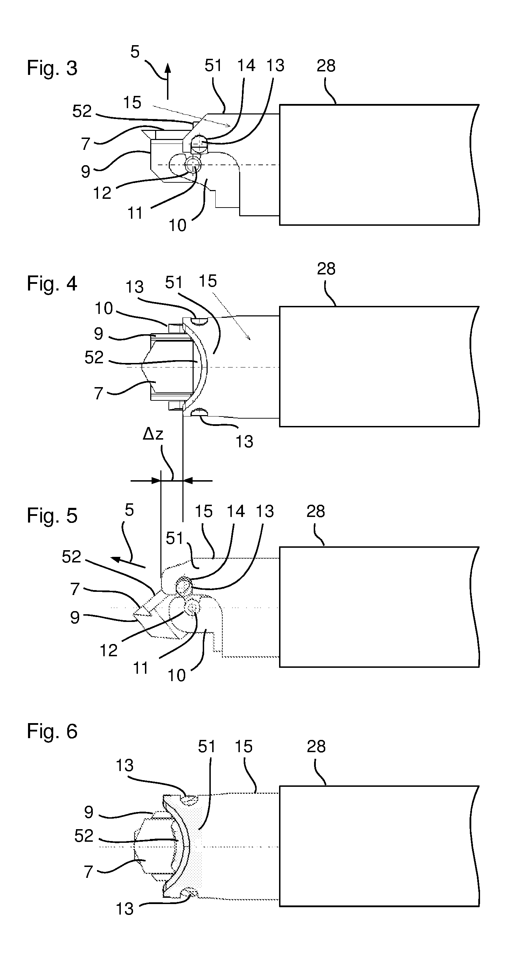

Die Abbildungsoptik 4 umfaßt ein Umlenkprisma 7 sowie diesem nachgeordnete Linsen 8. Das Umlenkprisma 7 sitzt in einem Prismenhalter 9, der schwenkbar am distalen Ende eines im Endoskopschaft 3 angeordneten Optikrohrs 10 gelagert ist, wie am besten in der vergrößerten Seitenansicht und Draufsicht des distalen Endes 6 des Endoskopschaftes 3 in Figuren 3 und 4 gezeigt ist, wobei bei den Darstellungen in Figuren 3 und 4 jeweils das Mantelrohr 29 nicht eingezeichnet ist.

Zur schwenkbaren Lagerung weist der Prismenhalter 9 zwei eine Schwenkachse bildende Lagerzapfen 11 auf, die in entsprechenden Aufnahmen 12 des distalen Endes des Optikrohrs 10 sitzen (in Fig. 3 sind nur der linke Lagerzapfen 11 sowie die linke Aufnahme 12 sichtbar).

Ferner weist der Prismenhalter 9 noch zwei Betätigungszapfen 13 auf, die in Aufnahmen 14 eines Zugrohrs 15 sitzen, in dem Optikrohr 10 positioniert ist. Das Zugrohr 15 ist in Längsrichtung des Endoskopschaftes 3 relativ zum Optikrohr 10 und zum Mantelrohr 29 verschiebbar gelagert, wobei die axiale Stellung des Zugrohrs 15 mittels eines am Handgriff 2 angeordneten Betätigungselementes 16 (Fig. 1) einstellbar ist, wie nachfolgend noch im Detail beschrieben wird.

In Fig. 5 und 6 sind die gleichen Ansichten wie in Fig. 3 und 4 gezeigt, wobei jedoch das Zugrohr 15 axial verschoben ist im Vergleich zu Fig. 3 und 4. Die axiale Verschiebung ist als Δz zwischen Figuren 4 und 5 eingezeichnet. Wie ein Vergleich der Darstellungen von Fig. 3 und 4 mit denen von Fig. 5 und 6 zeigt, führt eine axiale Verschiebung des Zugrohres 15 dazu, daß sich die Betätigungszapfen 13 mit dem konstruktiv vorgegebenen Abstand zu den Lagerzapfen 11 um diese bewegen. Die Betätigungszapfen 13 werden somit auf einer in der Zeichenebene von Fig. 3 und 5 liegenden Kreisbahn bewegt, deren Mittelpunkt der Lagerzapfen 11 und somit der Durchstoßpunkt der Schwenkachse mit der Zeichenebene von Fig. 3 und 5 ist. Diese Bewegung der Betätigungszapfen 13 ist möglich, da sie in den Aufnahmen 14 des distalen Endes des Zugrohrs 15 verschiebbar gelagert sind (in Fig. 3 und 5 gesehen, von oben nach unten verschiebbar).

Somit führt die axiale Verschiebung des Zugrohrs 15 zu einem Verschwenken des Prismenhalters 9 um die durch die Lagerzapfen 11 definierte Schwenkachse, die senkrecht zur Zeichenebene in Figuren 3 und 5 verläuft, und somit zu einem Verschwenken des Umlenkprismas 7, wodurch die Blickrichtung 5 der Abbildungsoptik 4 verändert wird. So ist die Blickrichtung 5 in der Darstellung von Fig. 3 und 4 ca. 90° (bezogen auf die Längsrichtung des Endoskopschaftes 3, die jeweils in der Zeichenebene von Figuren 3 bis 6 liegt und von links nach rechts verläuft). In Fig. 5 und 6 beträgt die Blickrichtung 5 hingegen ca. 10°.

Das Zugrohr 15 bzw. das distale Ende des Zugrohrs 15 ist so ausgebildet, daß es gleichzeitig als bewegliche Streulichtblende dient, die bei jeder Schwenkstellung des Umlenkprismas 7 sicher verhindert, daß unerwünschtes Streulicht in den Bereich 50 (Fig. 2) zwischen dem Prismenhalter 9 und den Linsen 8 der Abbildungsoptik 4 gelangt, was zu einer unerwünschten Verschlechterung der Abbildungsqualität der Abbildungsoptik 4 führen würde.

Dazu weist das Zugrohr 15 einen distalen oberen Abschirmungsabschnitt 51 auf, der stets direkt oberhalb des hinteren Teils 52 des Prismenhalters 9 positioniert ist, wie in Fig. 2 und auch in den verschiedenen Schwenkstellungen gemäß Fig. 3 bis 6 schematisch dargestellt ist. Dadurch wird gewährleistet, daß unabhängig von der eingestellten Schwenkstellung des Umlenkprismas 7 kein Streulicht in den Bereich 50 gelangt.

Das Zugrohr 15 dient somit zur Abschirmung des unerwünschten Streulichtes und treibt zeitgleich das Umlenkprisma 7 zur Einstellung der gewünschten Schwenkstellung und somit zur Einstellung der gewünschten Blickrichtung 5 an. So kann nach Einstellung einer neuen Schwenkstellung ohne Zeitverzögerung das Endoskop benutzt werden, da stets das Eindringen von Streulicht sicher verhindert wird. Die Abbildungsoptik 4 ist somit stets vor direkter Streulichteinstrahlung abgeschirmt.

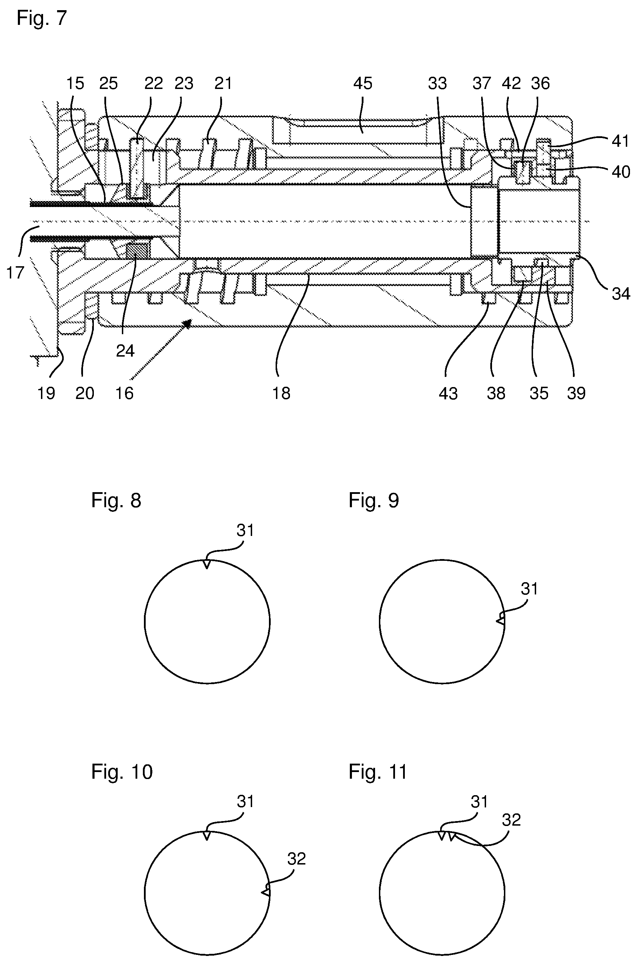

Die Abbildungsoptik 4 weist noch ein Bildübertragungssystem (hier in der Form von Stablinsen, von denen eine in der vergrößerten Schnittdarstellung des proximalen Abschnittes des Handgriffes 2 in Fig. 7 sichtbar ist) im Endoskopschaft 3 und im Handgriff 2 auf, das dazu dient, das aufgenommene Bild bis zum proximalen Ende des Handgriffes 2 zu übertragen, wo es dann bereitgestellt ist. Das bereitgestellte Bild kann direkt oder über ein proximal angeordnetes Okular betrachtet werden. Auch ist es möglich, beispielsweise eine Videokamera am proximalen Ende des Handgriffes 2 anzubringen, die das Bild aufnimmt und über eine Aufgabeeinheit (beispielsweise einen Monitor) darstellen kann.

Wie der vergrößerten Schnittdarstellung des proximalen Abschnitts des Handgriffs 2 in Fig. 7 zu entnehmen ist, ist das Betätigungselement 16 hülsenförmig ausgebildet und drehbar auf einer Führungshülse 18 gelagert, die ihrerseits drehfest mit einem Hauptteil 19 des Handgriffes 2 verbunden ist. Zwischen dem distalen Ende des Betätigungselements 16 und der Führungshülse 18 ist eine Gleitscheibe 20 vorgesehen.

Das Betätigungselement 16 weist auf seiner Innenseite im distalen Bereich eine erste schraubenförmig verlaufende Nut 21 auf, in die das obere Ende eines ersten Bolzens 22 einsteht. Der erste Bolzen 22 erstreckt sich dabei durch eine in Längsrichtung des Endoskopschaftes 3 (und somit von rechts nach links in Fig. 7) erstreckendes erstes Langloch 23 der Führungshülse 18 und ist mit seinem unteren Ende über einen ersten Teflonring 24 in einem Verbindungsteil 25 befestigt, das mit dem proximalen Ende des Zugrohrs 15 drehfest verbunden ist.

Aufgrund dieses Aufbaus führt eine Drehung des Betätigungselementes 16 um die Längsachse des Endoskopes und relativ zum Hauptteil 19 dazu, daß der erste Bolzen 22 in axialer Richtung (aufgrund der Führung durch das erste Langloch 23 in der drehfest mit dem Hauptteil 19 verbundenen Führungshülse 18) bewegt wird, so daß das Verbindungsteil 25 und somit das Zugrohr 15 axial verschoben werden. Diese Verschiebung führt am distalen Ende des Zugrohres 15, wie in Fig. 3 bis 6 dargestellt ist, dazu, daß eine gewünschte Schwenkstellung des Umlenkprismas 7 eingestellt wird.

Der Handgriff 2 umfaßt ferner ein Mittelteil 26 mit einem Lichtleiteranschluß 27 (Fig. 1). Das Mittelteil 26 ist drehfest mit dem Endoskopschaft 3 verbunden und kann relativ zum Hauptteil 19 um die Längsachse des Endoskopschaftes 3 gedreht werden. Dazu ist der Endoskopschaft 3 drehbar im Hauptteil 19 geführt.

Ferner umfaßt der Endoskopschaft 3, wie am besten aus Fig. 2 ersichtlich ist, ein Innenrohr 28, in dem das Zugrohr 15, das Optikrohr 10 sowie die Abbildungsoptik 4 angeordnet sind, und das Mantelrohr 29, in dem das Innenrohr 28 eingesetzt ist. Der Innendurchmesser des Mantelrohrs 29 ist größer als der Außendurchmesser des Innenrohrs 28, so daß zwischen den beiden Rohren 28 und 29 ein sich entlang der Längsrichtung des Endoskopschaftes 3 erstreckender Zwischenraum 30 vorhanden ist. In dem Zwischenraum 30 sind (in Fig. 2 nicht gezeigte) Lichtleitfasern angeordnet, die zur Beleuchtung des abzubildenden Objektes dienen. Die Lichtleitfasern können über den Lichtleiteranschluß 27 am Mittelteil 26 mit Licht beaufschlagt werden. Das Mantelrohr 29 weist eine distale Öffnung 48 auf, die mittels einer Glasabdeckung 49 verschlossen ist (bevorzugt hermetisch dicht), so daß die Abbildungsoptik 4 vor Verschmutzungen geschützt ist.

Damit der Benutzer die Drehstellung des Endoskopschaftes optisch erfassen kann, wenn er das mittels der Abbildungsoptik 4 erzeugte Bild betrachtet, ist am distalen Ende des Optikrohrs (Fig. 7) eine feste Markierungshülse 33 mit einer festen Markierungsblende 31 drehfest mit dem Optikrohr 10 verbunden. Die feste Markierungsblende 31 weist hier die Form eines Dreieckes auf, wie in Fig. 8 schematisch dargestellt ist. Wenn der Benutzer den Endoskopschaft um 90° nach rechts dreht (durch Drehen des Mittelteiles 26 relativ zum Hauptteil 19), bewegt sich die feste Markierungshülse 33 und somit die feste Markierungsblende 31 mit, so daß der Benutzer die Markierungsblende in der in Fig. 9 gezeigten Position zusammen mit dem Bild sieht.

Bei der hier beschriebenen Ausführungsform des erfindungsgemäßen Endoskop ist jedoch nicht nur eine feste Markierungsblende 31 vorgesehen, sondern zusätzlich noch eine drehbare Markierungsblende 32, die im Bild ferner die Blickrichtung 5 der Abbildungsoptik 4 und somit die Schwenkstellung des Umlenkprismas 7 anzeigt. Die drehbare Markierungsblende 32 kann die gleiche Form (beispielsweise Dreiecksform) wie die feste Markierungsblende 31 aufweisen, wie in Fig. 10 schematisch angedeutet ist. In Fig. 10 ist der Fall gezeigt, daß die Schwenkstellung des Prismas ca. 90° entspricht. Wird nun die in Fig. 5 und 6 gezeigte Schwenkstellung eingestellt (Blickrichtung von ca. 10°), ändert sich die Position der drehbaren Markierungsblende 32 relativ zur festen Markierungsblende 31, wie in Fig. 11 dargestellt ist. Somit sieht der Benutzer bei Betrachtung des Bildes über den Drehwinkel zwischen beiden Markierungsblenden 31 und 32 die eingestellte Schwenkstellung des Umlenkprismas und daher die eingestellte Blickrichtung 5. Natürlich sind die beiden Markierungsblenden 31 und 32 bevorzugt so ausgebildet, daß sie eindeutig unterscheidbar sind. So können die beiden Markierungsblenden 31 und 32 unterschiedliche Farben aufweisen. Zusätzlich oder alternativ können die beiden Markierungsblenden 31, 32 unterschiedliche Formen besitzen.

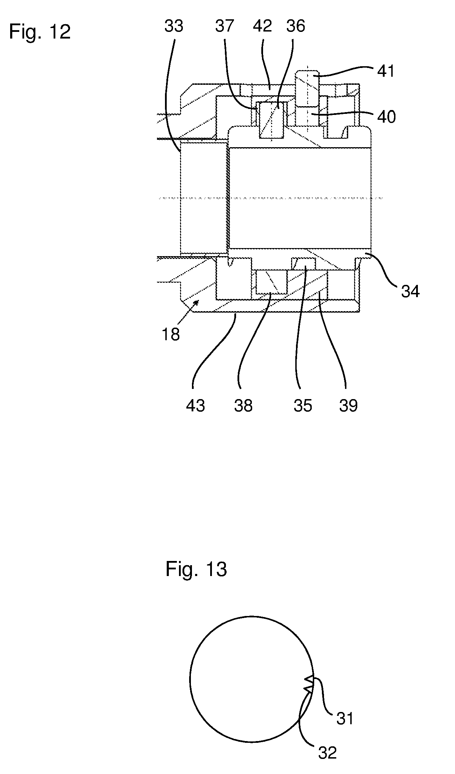

Die drehbare Markierungsblende 32 ist auf der Innenseite einer drehbaren Markierungshülse 34 (Fig. 7) so befestigt, so daß sie im Sichtfeld des dargestellten Bildes liegt. Die drehbare Markierungshülse 34 weist auf ihrer Außenseite eine schraubenförmig verlaufende Nut 35 auf, in die ein erster Stift 36 einsteht, wie aus Fig. 7 sowie aus der vergrößerten Detaildarstellung in Fig. 12 ersichtlich ist.

Der erste Stift 36 ist fest mit einem Teflonring 37 verbunden, der drehbar in einer ringförmigen Innennut 38 einer Gleithülse 39 sitzt. Die Gleithülse 39 weist axial zur Innennut 38 beabstandet eine Bohrung 40 auf, in der ein zweiter Stift 41 sitzt, der durch ein zweites Langloch 42 der Führungshülse 18 hindurchsteht, das sich in Längsrichtung des Endoskopschaftes 3 erstreckt. Das obere Ende des zweiten Stiftes 41 mündet in einer Nut 43, die auf der Innenseite des Betätigungselementes 16 ausgebildet ist und sich im proximalen Bereich des Betätigungselementes 16 schraubenförmig erstreckt.

Aufgrund dieses Aufbaus führt eine Drehung des Betätigungselementes 16 relativ zur Führungshülse 18 dazu, daß die Drehbewegung des Betätigungselementes 16 in eine axiale Bewegung des zweiten Stiftes 41 aufgrund des zweiten Langloches 42 umgesetzt wird. Die axiale Bewegung des zweiten Stiftes 41 führt zu einer axialen Bewegung der Gleithülse 39, was aufgrund des ersten Stiftes 36 dann zu einer Drehbewegung der drehbaren Markierungshülse 34 führt. Somit wird synchron mit dem Verschwenken des Umlenkprismas 7 aufgrund einer Drehung des Betätigungselementes 16 die drehbare Markierungshülse 34 und somit die drehbare Markierungsblende 32 relativ zum Optikrohr 10 und somit relativ zur festen Markierungsblende 31 gedreht.

Wenn der Benutzer im Vergleich zu der in Fig. 11 gezeigten Stellung den Endoskopschaft 3 um 90° nach rechts dreht (durch Drehen des Mittelteils 26 relativ zum Hauptteil 19), wird abei auch das Zugrohr 15 um 90° nach rechts gedreht, wodurch über den Bolzen 22 das Betätigungselement 16 ebenfalls nach rechts gedreht wird. Dies führt zu einer Drehung der drehbaren Markierungshülse 34 um 90° nach rechts. Da die feste Markierungshülse 33 aufgrund der Drehung des Endoskopschaftes 3 ebenfalls um 90° nach rechts gedreht wird, sieht ein Benutzer beim Betrachten des Bildes die beiden Markierungsblenden 31, 32 in der in Fig. 13 gezeigten Stellung. Der Benutzer kann somit unmittelbar die Drehstellung des Endoskopschaftes von 90° und die Blickrichtung von 10° erfassen.

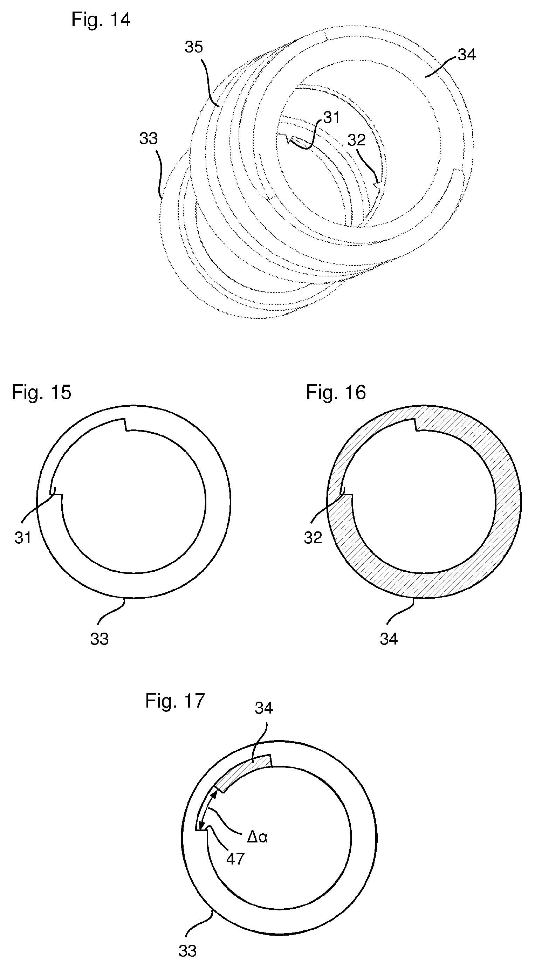

Die beiden Markierungshülsen 33 und 34 sind in Fig. 14 vergrößert in einer perspektivischen Explosionsdarstellung gezeigt. In dieser Darstellung sind die Markierungsblenden 31, 32 deutlich sichtbar.

Die gesamte Mechanik zur Drehung der drehbaren Markierungsblende 32 ist hier als Untersetzungsgetriebe ausgelegt, so daß eine Drehung des Betätigungselementes 16 um einen vorbestimmten Drehwinkel zu einer Drehung der drehbaren Markierungsblende 32 führt, bei der der Drehwinkel kleiner ist als der vorbestimmte Drehwinkel. Die Ausbildung als Untersetzungsgetriebe ist hier gewählt, da die benötigte axiale Verschiebung des Zugrohrs 15 zum Verschwenken des Umlenkprismas 7 einen Drehwinkel des Betätigungselementes 16 erfordert, der größer ist als der gewünschte maximale Drehwinkel der drehbaren Markierungsblende 32 bzw. der drehbaren Markierungshülse 34.

Damit ist es möglich, die Schwenkstellung des Prismas 7 sehr genau einzustellen, da ein relativ großer Drehwinkel des Betätigungselementes 16 notwendig ist, um das Umlenkprisma um einen vorbestimmten Winkel zu verschwenken. Da das Untersetzungsgetriebe so ausgelegt, daß der Winkelabstand der beiden Markierungsblenden 31 und 32 gerade dem Winkel der eingestellten Blickrichtung 5 entspricht, kann trotzdem eine für den Benutzer sinnvolle Darstellung erreicht werden.

Die Außenseite des Betätigungselementes 16 kann ergonomisch ausgebildet sein. So sind in der Darstellung von Fig. 1 als auch in der Darstellung von Fig. 7 Vertiefungen 45 sichtbar, die einen guten Halt gewährleisten.

Wie bereits erwähnt, können die Markierungsblenden 31 und 32 auch andere Formen aufweisen. In Fig. 15 ist eine Draufsicht einer Variante der festen Markierungshülse 33 gezeigt. Die ringabschnittsförmige Ausnehmung dient als feste Markierungsblende 31. In Fig. 16 ist eine Draufsicht auf eine Variante der drehbaren Markierungshülse 34 gezeigt, wobei in gleicher Weise wie bei der festen Markierungshülse 33 die ringabschnittsförmige Ausnehmung die drehbare Markierungsblende 32 bildet. Um die beiden Markierungshülsen in der Darstellung unterscheiden zu können, ist die drehbare Markierungshülse 34 schraffiert dargestellt. Aufgrund der beschriebenen Anordnung der beiden Markierungshülsen 33 und 34 führt eine Verdrehung der drehbaren Markierungshülse 34 relativ zur festen Markierungshülse 33 dazu, daß der Winkelabstand Δα zwischen beiden ringabschnittförmigen Ausnehmungen bzw. zwischen beiden Markierungsblenden 31 und 32 sich ändert (wie in Fig. 17 angedeutet ist). Dieser Winkelabstand Δα zeigt dann für den Betrachter im Bild wiederum die Schwenkstellung des Umlenkprismas 7 an. Die untere Kante 47 zeigt z.B. die Drehstellung des Endoskopschaftes 3 an.

## Patentansprüche

1. Endoskop mit
einem Handgriff (2),
einem mit dem Handgriff (2) verbundenen Endoskopschaft (3),
einer im Endoskopschaft (3) angeordneten Abbildungsoptik (4), die ein in Blickrichtung (5) der Abbildungsoptik (4) vor dem Endoskopschaft (3) befindliches Objekt als Bild abbildet und die ein zur Einstellung der Blickrichtung (5) schwenkbar gelagertes Umlenkelement (7) aufweist, das am vom Handgriff (2) abgewandten distalen Ende des Endoskopschaftes (3) angeordnet ist,
sowie mit einem am Handgriff (2) angeordneten Betätigungselement (16), das mit dem Umlenkelement (7) mechanisch gekoppelt ist und mit dem die Schwenkstellung des Umlenkelementes (7) geändert werden kann, um eine gewünschte Blickrichtung (5) einzustellen,
wobei ein Schiebeelement (15), dessen proximales Ende mit dem Betätigungselement (16) gekoppelt
ist und dessen distales Ende einen Abschirmungsabschnitt (51) aufweist, verschiebbar im Endoskopschaft (3) angeordnet ist,
wobei eine Betätigung des Betätigungselementes (16) zu einem Verschieben des Schiebeelementes (15) führt und
wobei das distale Ende des Schiebeelementes (15) so mit dem Umlenkelement (7) gekoppelt ist, daß eine Verschiebung des Schiebeelements (15) eine Änderung der Schwenkstellung des Umlenkelementes (7) und gleichzeitig eine Verschiebung des Abschirmungsabschnittes (51) bewirkt, um zeitgleich mit der Einstellung der gewünschten Blickrichtung eine Streulichtabschirmung für den Bereich (50) zwischen dem Umlenkelement (7) und einem dem Umlenkelement nachfolgenden Optikelement (8) der Abbildungsoptik (4) zu erreichen, **dadurch gekennzeichnet, daß** das Schiebeelement (15) als Rohr ausgebildet ist.

2. Endoskop nach Anspruch 1, **dadurch gekennzeichnet, daß** das distale Ende des Schiebeelementes (15) zum Verschwenken des Umlenkelementes (7) an einem Bereich angreift, der nicht auf der Schwenkachse des Umlenkelementes (7) liegt.

3. Endoskop nach einem der obigen Ansprüche, **dadurch gekennzeichnet, daß** zur Kopplung des distalen Endes des Schiebeelementes (15) mit dem Umlenkelement (7) ein Betätigungszapfen (13) als erstes Verbindungselement und eine den Betätigungszapfen (13) führende Aufnahme (14) als zweites Verbindungselement vorgesehen sind, wobei eines der beiden Verbindungselemente am Umlenkelement (7) und das andere der beiden Verbindungselemente am Schiebeelement (15) vorgesehen ist.

4. Endoskop nach einem der obigen Ansprüche, **dadurch gekennzeichnet, daß** das Betätigungselement (16) drehbar am Handgriff (2) gelagert ist und so mit dem Schiebeelement (15) gekoppelt ist, daß eine Drehung des Betätigungselementes (16) zu einer Verschiebung des Schiebeelementes (15) führt.

5. Endoskop nach einem der obigen Ansprüche, **dadurch gekennzeichnet, daß** eine erste Markierungsblende (32) vorgesehen ist, die mechanisch mit dem Betätigungselement (16) so gekoppelt ist, daß sie synchron mit einer Änderung der Schwenkstellung des Umlenkelementes (7) bewegt wird und mittels ihrer sichtbaren Lage bei Betrachtung des Bildes die eingestellte Blickrichtung (5) anzeigt.

6. Endoskop nach einem der obigen Ansprüche, **dadurch gekennzeichnet, daß** der Endoskopschaft (3) drehbar im Handgriff (2) gelagert ist und eine zweite Markierungsblende (31) aufweist, die drehfest mit dem Endoskopschaft (3) verbunden ist und die mittels ihrer sichtbaren Lage bei Betrachtung des Bildes die Drehstellung des Endoskopschaftes anzeigt.

7. Endoskop nach Anspruch 6, **dadurch gekennzeichnet, daß** die Lage der ersten Markierungsblende (32) relativ zur Lage der zweiten Markierungsblende (31) bei Betrachtung des Bildes die eingestellte Blickrichtung (5) anzeigt.

8. Endoskop nach Anspruch 7, **dadurch gekennzeichnet, daß** bei Bewegung der ersten Markierungsblende (32) ihr Winkelabstand zur zweiten Markierungsblende (31) verändert wird, um mittels des Winkelabstandes die Blickrichtung (5) anzuzeigen.

9. Endoskop nach einem der obigen Ansprüche, **dadurch gekennzeichnet, daß** das Betätigungselement (16) mit der ersten Markierungsblende (32) über ein Untersetzungsgetriebe verbunden ist.

10. Endoskop nach Anspruch 9, **dadurch gekennzeichnet, daß** das Untersetzungsgetriebe eine Drehung des Betätigungselementes (16) in eine axiale Bewegung und die axiale Bewegung in eine Drehbewegung der ersten Markierungsblende (32) umsetzt.

11. Endoskop nach einem der obigen Ansprüche, **dadurch gekennzeichnet, daß** die erste Markierungsblende (32) im Bereich der Feldblende der Abbildungsoptik (4) positioniert ist.

12. Endoskop nach einem der obigen Ansprüche, **dadurch gekennzeichnet, daß** die erste Markierungsblende (32) relativ zur Abbildungsoptik (4) drehbar gelagert ist.

## Claims

1. An endoscope, comprising
a handle (2),
an endoscope shaft (3) connected to the handle (2),
imaging optics (4) arranged in the endoscope shaft (3) with which an object located in the direction of view (5) of the imaging optics (4) in front of the endoscope shaft (3) is imaged as an image and which has a swivellably housed deflecting element (7) to set the desired direction of view (5), said deflecting element is positioned at the distal end of the endoscope shaft (3) facing away from the handle (2), and an actuating element (16) attached to the handle (2) which is mechanically connected to the deflecting element (7) and with which the swivel position of the deflecting element (7) can be changed in order to set a desired direction of view (5),
wherein a sliding element (15), the proximal end of which is connected to the actuating element (16) and the distal end of which has a screening section (51), is arranged displaceable in the endoscope shaft (3),
wherein an actuation of the actuating element (16) leads to a displacement of the sliding element (15), and
wherein the distal end of the sliding element (15) is connected to the deflecting element (7) such that a displacement of the sliding element (15) brings about a change in the swivel position of the deflecting element (7) and simultaneously a displacement of the screening section (51), in order to achieve, at the same time as setting the desired direction of view, a scattered-light screening for the area (50) between the deflecting element (7) and an optical element (8) following the deflecting element of the imaging optics (4), **characterized in that** the sliding element (15) is configured as a tube.

2. The endoscope according to claim 1, wherein, to swivel the deflecting element (7), the distal end of the sliding element (15) engages in an area which does not lie on the swivel axis of the deflecting element (7).

3. The endoscope according to one of the above claims, wherein, to connect the distal end of the sliding element (15) to the deflecting element (7) an actuating pin (13) is provided as a first connecting element and a receptacle (14) guiding the actuating pin (13) as a second connecting element, wherein one of the two connecting elements is provided at the deflecting element (7) and the other of the two connecting elements at the sliding element (15).

4. The endoscope according to one of the above claims, wherein the actuating element (16) is rotatably housed on the handle (2) and is connected to the sliding element (15) such that a rotation of the actuating element (16) leads to a displacement of the sliding element (15).

5. The endoscope according to one of the above claims, wherein a first marking stop (32) is provided which is mechanically connected to the actuating element (16) such that it is moved synchronously with a change in the swivel position of the deflecting element (7) and displays the set direction of view (5) by means of its visible position when observing the image.

6. The endoscope according to one of the above claims, wherein the endoscope shaft (3) is rotatably housed on the handle (2) and has a second marking stop (31) which is connected in rotation-resistant manner to the endoscope shaft (3) and which displays the rotation position of the endoscope shaft by means of its visible position when observing the image.

7. The endoscope according to claim 6, wherein the position of the first marking stop (32) relative to the position of the second marking stop (31) displays the set direction of view (5) when observing the image.

8. The endoscope according to claim 7, wherein, when the first marking stop (32) moves, its angular separation from the second marking stop (31) is changed in order to display the direction of view (5) by means of the angular separation.

9. The endoscope according to one of the above claims, wherein the actuating element (16) is connected to the first marking stop (32) via a reduction gear mechanism.

10. The endoscope according to claim 9, wherein the reduction gear mechanism converts a rotation of the actuating element (16) into an axial movement and the axial movement into a rotational movement of the first marking stop (32).

11. The endoscope according to one of the above claims, wherein the first marking stop (32) is positioned in the area of the field stop of the imaging optics (4).

12. The endoscope according to one of the above claims, wherein the first marking stop (32) is rotatably housed relative to the imaging optics (4).

## Revendications

1. Endoscope comportant :
une poignée (2),
une tige d'endoscope (3) reliée à la poignée (2),
une optique de formation d'image (4) disposée dans la tige d'endoscope (3), laquelle optique forme l'image d'un objet se trouvant dans la direction d'observation (5) de l'optique de formation d'image (4) à l'avant de la tige d'endoscope (3) et comporte un élément de déviation (7) monté pivotant pour régler la direction d'observation (5), lequel élément de déviation est disposé sur l'extrémité distale de la tige d'endoscope (3) qui est opposée à la poignée (2),
ainsi qu'un élément d'actionnement (16) disposé sur la poignée (2), lequel élément d'actionnement est mécaniquement couplé à l'élément de déviation (7) et au moyen duquel la position de pivotement de l'élément de déviation (7) peut être modifiée afin de régler une direction d'observation (5) souhaitée,
dans lequel un élément coulissant (15), dont l'extrémité proximale est couplée à l'élément d'actionnement (16) et dont l'extrémité distale présente une section d'occultation (51), est disposé de manière coulissante dans la tige d'endoscope (3),
dans lequel un actionnement de l'élément d'actionnement (16) conduit à un coulissement de l'élément coulissant (15), et
dans lequel l'extrémité distale de l'élément coulissant (15) est couplée à l'élément de déviation (7) de manière à ce qu'un coulissement de l'élément coulissant (15) provoque une modification de la position de pivotement de l'élément de déviation (7) et un coulissement simultané de la section d'occultation (51), afin d'occulter simultanément la lumière parasite grâce au réglage de la direction d'observation souhaitée pour la région (50) située entre l'élément de déviation (7) et un élément optique (8) de l'optique de formation d'image (4) monté en aval de l'élément de déviation,
**caractérisé en ce que** l'élément coulissant (15) est réalisé sous la forme d'un tube.

2. Endoscope selon la revendication 1, **caractérisé en ce que** l'extrémité distale de l'élément coulissant (15) destiné à faire pivoter l'élément de déviation (7) est en prise avec une région qui ne se situe pas sur l'axe de pivotement de l'élément de déviation (7).

3. Endoscope selon l'une quelconque des revendications précédentes, **caractérisé en ce que**, pour coupler l'extrémité distale de l'élément coulissant (15) à l'élément de déviation (7), il est prévu un doigt d'actionnement (13) en tant que premier élément de liaison et un évidement (14) guidant le doigt d'actionnement (13) en tant que second élément de liaison, dans lequel l'un des deux éléments de liaison est prévu sur l'élément de déviation (7) et l'autre des deux éléments de liaison est prévu sur l'élément coulissant (15).

4. Endoscope selon l'une quelconque des revendications précédentes, **caractérisé en ce que** l'élément d'actionnement (16) est monté de manière rotative sur la poignée (2) et est couplé à l'élément coulissant (15) de manière à ce qu'une rotation de l'élément d'actionnement (16) conduise à un coulissement de l'élément coulissant (15).

5. Endoscope selon l'une quelconque des revendications précédentes, **caractérisé en ce qu'**il est prévu un premier obturateur de marquage (32) qui est mécaniquement couplé à l'élément d'actionnement (16) de manière à ce qu'il se déplace en synchronisme avec une modification de la position de pivotement de l'élément de déviation (7) et indique par sa position visible lors du visionnage de l'image la direction d'observation (5) réglée.

6. Endoscope selon l'une quelconque des revendications précédentes, **caractérisé en ce que** la tige d'endoscope (3) est montée de manière rotative dans la poignée (2) et comporte un second obturateur de marquage (31) qui est relié de manière solidaire en rotation à la tige d'endoscope (3) et qui indique par sa position visible lors du visionnage de l'image la position de rotation de la tige d'endoscope.

7. Endoscope selon la revendication 6, **caractérisé en ce que** la position du premier obturateur de marquage (32) par rapport à la position du second obturateur de marquage (31) lors du visionnage de l'image indique la direction d'observation (5) réglée.

8. Endoscope selon la revendication 7, **caractérisé en ce que**, lors d'un déplacement du premier obturateur de marquage (32), sa distance angulaire par rapport au second obturateur de marquage (31) est modifiée afin d'indiquer la direction d'observation (5) au moyen de la distance angulaire.

9. Endoscope selon l'une quelconque des revendications précédentes, **caractérisé en ce que** l'élément d'actionnement (16) est relié au premier obturateur de marquage (32) par l'intermédiaire d'un réducteur de vitesse.

10. Endoscope selon la revendication 9, **caractérisé en ce que** le réducteur de vitesse convertit une rotation de l'élément d'actionnement (16) en un mouvement axial et convertit le mouvement axial en un mouvement de rotation du premier obturateur de marquage (32).

11. Endoscope selon l'une quelconque des revendications précédentes, **caractérisé en ce que** le premier obturateur de marquage (32) est positionné dans la région de l'obturateur de champ de l'optique de formation d'image (4).

12. Endoscope selon l'une quelconque des revendications précédentes, **caractérisé en ce que** le premier obturateur de marquage (32) est monté de manière rotative par rapport à l'optique de formation d'image (4).
